(19) Europäisches Patentamt
European Patent Office
Office européen des brevets

(11) EP 1 812 366 B1

(12) **EUROPÄISCHE PATENTSCHRIFT**

(45) Veröffentlichungstag und Bekanntmachung des Hinweises auf die Patenterteilung:
**11.11.2009 Patentblatt 2009/46**

(21) Anmeldenummer: **05813591.4**

(22) Anmeldetag: **16.11.2005**

(51) Int Cl.:
***C07C 29/145*** *(2006.01)* ***B01J 23/72*** *(2006.01)*
***B01J 23/26*** *(2006.01)*

(86) Internationale Anmeldenummer:
**PCT/EP2005/012294**

(87) Internationale Veröffentlichungsnummer:
**WO 2006/053735 (26.05.2006 Gazette 2006/21)**

(54) **VERFAHREN ZUR HYDRIERUNG VON KETONEN**

METHOD FOR THE HYDROGENATION OF KETONES

PROCEDE D'HYDROGENATION DE CETONES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **16.11.2004 DE 102004055189**

(43) Veröffentlichungstag der Anmeldung:
**01.08.2007 Patentblatt 2007/31**

(73) Patentinhaber: **SÜD-CHEMIE AG**
**80333 München (DE)**

(72) Erfinder: **LADEBECK, Jürgen**
**Louisville, KY 40232 (US)**

(74) Vertreter: **Dannenberger, Oliver Andre**
**Abitz & Partner**
**Patentanwälte**
**Postfach 86 01 09**
**81628 München (DE)**

(56) Entgegenhaltungen:
**GB-A- 587 181 US-A- 5 124 295**

- **D. NIGHTINGALE AND H. D. RADFORD: "The hydrogenation of aromatic ketones with hydrogen and copper-chromium oxide catalyst" JOURNAL OF ORGANIC CHEMISTRY, Bd. 14, 1949, Seiten 1089-1093, XP002365585**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 812 366 B1

Printed by Jouve, 75001 PARIS (FR)

## Beschreibung

Die Erfindung betrifft ein Verfahren zur Hydrierung von Ketonen.

Die Hydrierung von Acetophenon (Methylphenylketon = MPK) zu Methylphenylcarbinol (MPC) nach dem nachstehend angegebenen Reaktionsschema ist ein wichtiger industrieller Schritt bei der Herstellung von Styrol; sie geht gewöhnlich mit der Herstellung von Propylenoxid durch Oxidation einher (Oxiranprozess).

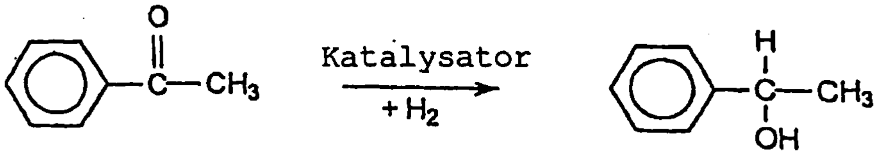


Das aus MPC durch Dehydratisierung erhaltene Styrol kann leicht abgetrennt werden und steht in einer viel größeren Reinheit zur Verfügung als Styrol, das durch direkte Dehydrierung von Ethylbenzol erhalten wird. Die Hydrierung von Acetophenon eröffnet auch einen interessanten wirtschaftlichen Weg in Fällen, in denen Acetophenon als Nebenprodukt erhalten wird. Der Markt für Acetophenon ist auf einige Spezialanwendungen beschränkt, während Styrol vom Markt leicht aufgenommen wird. In technischen Systemen, wie sie beispielsweise beim Oxiranprozeß verwendet werden, kann das Acetophenon entweder in einer dispergierten Phase oder in einem Festbett hydriert werden. Die verwendeten Katalysatoren sind üblicherweise Kupferchromite oder Kupfer-Zink-Chromite, die eine relativ hohe MPC-Selektivität zeigen.

Ein Verfahren zur Hydrierung von aromatischen Ketonen (z. B. Acetophenon) mit Wasserstoff in gegenwart eines Kupferchromit-Katalysators wird in Journal of Organic Chemistry 1949, 14 1089-1093 beschrieben.

Der Erfindung liegt die Aufgabe zu Grunde, die Hydrieraktivität sowie die Selektivität bei der Hydrierung von Ketonen noch weiter zu erhöhen.

Es wurde überraschenderweise gefunden, dass diese Aufgabe mit Hilfe von $SiO_2$-haltigen Kupferchromit-Katalysatoren gelöst werden kann.

Gegenstand der Erfindung ist somit ein Verfahren zur Hydrierung von Ketonen, wobei das Keton im Gemisch mit Wasserstoff einem Katalysatorbett zugeführt wird, das einen Kupferchromitkatalysator aufweist, welcher einen Anteil an $SiO_2$ enthält.

Es hat sich gezeigt, dass der Katalysator durch den Zusatz von $SiO_2$ eine höhere Stabilität erreicht und daher die Standzeit des Katalysators wesentlich verlängert werden kann. Ferner zeigt der im erfindungsgemäßen Verfahren eingesetzte Kupferchromitkatalysator eine geringere Neigung zur Verkupferung, d.h. die für die katalysierte Reaktion zur Verfügung stehende Kupferfläche verringert sich während der Lebenszeit des Katalysators in deutlich geringerem Ausmaß als bei herkömmlich verwendeten Kupferchromitkatalysatoren. Dies bedeutet, dass über die Betriebszeit hinweg eine geringere Abnahme der Aktivität des Katalysators erreicht wird. Schließlich zeigt der im erfindungsgemäßen Verfahren eingesetzte Katalysator eine deutlich höhere Hydrieraktivität im Vergleich zu bisher für die Hydrierung von Ketonen eingesetzten Hydrierkatalysatoren. Ein weiterer Vorteil des erfindungsgemäßen Verfahrens liegt darin, dass der eingesetzte Katalysator eine erhöhte Stabilität aufweist. Während des Einsatzes in einem Reaktor zerfällt der Katalysator daher sehr langsam, so dass lange Betriebszeiten erreicht werden, bis ein Austausch der Reaktorfüllung erforderlich wird.

Der Anteil an $SiO_2$ am Kupferchromitkatalysator beträgt, bezogen auf den oxidierten Katalysator, 5 bis 15 Gew.-%, insbesondere bevorzugt 6 bis 11 Gew.-%.

Der Katalysator enthält Kupfer in einem Anteil von 30 bis 40 Gew.-%, insbesondere 33 bis 39 Gew.-% sowie Chrom in einem Anteil von 20 bis 30 Gew.-%, vorzugsweise 24 bis 29 Gew.-%. Die prozentualen Anteile beziehen sich auf die oxidierte Form des Katalysators.

Der im erfindungsgemäßen Verfahren eingesetzte Katalysator kann auch Promotormetalle enthalten, um die Eigenschaften des Katalysators zu beeinflussen. Die Promotermetalle werden dabei in einem Anteil von 1 bis 6 Gew.-%, insbesondere bevorzugt 1,5 bis 5 Gew.-% zugegeben. Die Promotormetalle sind dabei vorzugsweise ausgewählt aus Barium und/oder Mangan.

Vorzugsweise enthält der Katalysator neben den oben genannten Metallen Kupfer und Chrom sowie ggf. Barium und/oder Mangan, welche als Oxid bzw. in einer in ein Oxid überführbaren Form vorliegen können, und dem $SiO_2$ weitere Metalle nur in geringen Mengen. Derartige Metalle sind beispielsweise Zink, Eisen oder Aluminium. Der Anteil dieser weiteren Metalle, bezogen auf den oxidierten Katalysator, beträgt in der Summe vorzugsweise weniger als 1 Gew.-%, besonders bevorzugt weniger als 0,5 Gew.-%, insbesondere bevorzugt weniger als 0,4 Gew.-%. Der Anteil jedes einzelnen weiteren Metalls beträgt, bezogen auf den oxidierten Katalysator, vorzugsweise weniger als 0,2 Gew.-

%, insbesondere bevorzugt weniger als 0,1 Gew.-%. Insbesondere bevorzugt beträgt der Anteil an Eisen, Zink und Aluminium jeweils weniger als 0,1 Gew.-%.

Die im erfindungsgemäßen Verfahren eingesetzten Kupferchromitkatalysatoren weisen vorzugsweise eine spezifische Oberfläche von etwa 50 bis 80 $m^2/g$, insbesondere von etwa 60 bis 70 $m^2/g$ auf. Das Porenvolumen der eingesetzten Katalysatoren liegt vorzugsweise im Bereich von 120 bis 200 $mm^3/g$, insbesondere bevorzugt 140 bis 180 $mm^3/g$.

Das Katalysatorbett ist vorzugsweise als Festbett ausgestaltet. Die Katalysatoren liegen bei der Festbetthydrierung im allgemeinen als Formkörper, z.B. als Tabletten, vor. Eine besonders günstige Tablettengröße beträgt etwa 3x3 mm. An sich unterliegt die Form der Formkörper jedoch keinen Beschränkungen, so dass neben einer Ausgestaltung als Tablette auch andere Formen verwendet werden können. Die Formkörper haben im Allgemeinen eine Schüttdichte von 1400 bis 1500 g pro Liter, eine Seitendruckfestigkeit von etwa 70 bis 105 N und ein Porenvolumen von etwa 160 bis 220 $mm^3/g$.

Die im erfindungsgemäßen Verfahren verwendeten Katalysatoren sind im Handel erhältlich. So wird von der Süd-Chemie AG unter der Bezeichnung "G-22/2" ein Katalysator angeboten, welcher im erfindungsgemäßen Verfahren verwendet werden kann. Der o.g. Katalysator wurde bisher nur für die Hydrierung von Aldehyden verwendet. Überraschend wurde nun gefunden, dass dieser Katalysator auch für die Hydrierung von Ketonen eingesetzt werden kann, wobei hohe Ausbeuten bei einer hohen Selektivität erreicht werden, so dass sich der Katalysator insbesondere auch für eine großtechnische Hydrierung von Ketonen eignet.

Der Katalysator kann nach an sich bekannten Verfahren hergestellt werden. So kann der im erfindungsgemäßen Verfahren eingesetzte Katalysator beispielsweise durch Fällung hergestellt werden. Dazu werden zunächst wässrige Lösungen der Metallsalze hergestellt, beispielsweise in Form von Chromaten oder Nitraten, und kontrolliert vereinigt, so dass ein Niederschlag erhalten wird. Zu zumindest einer der Metallsalzlösungen wird $SiO_2$ zugegeben. Der Niederschlag wird mit üblichen Verfahren abgetrennt, getrocknet und gegebenenfalls calciniert. Für die Herstellung eines Festbetts wird der Katalysator dann in üblicher Weise zu Formkörpern gepresst. Dazu können die üblichen Hilfsmittel zugemischt werden, beispielsweise Graphit als Schmermittel. Für die Herstellung der Tabletten können übliche Tablettenpressen verwendet werden.

Das für die Herstellung des Katalysators geschilderte Verfahren ist nur als Beispiel aufzufassen. Es ist auch möglich, Kupferchromitkatalysatoren, welche einen Anteil an $SiO_2$ enthalten, nach anderen Verfahren herzustellen. Derartige Verfahren sind dem Fachmann bekannt, so dass er die Herstellung des Katalysators ohne weiteres durch übliche Ermittlung der Herstellungsparameter erfolgen kann.

Das erfindungsgemäße Verfahren wird vorzugsweise mit einem Festbettkatalysator durchgeführt. Der Katalysator wird dazu in üblicher Weise in einen Festbettreaktor eingefüllt. Um eine gleichmäßige Strömung bzw. Temperaturverteilung zu erreichen, kann vor dem Katalysatorbett eine Zone angeordnet sein, welche aus einem inaktiven Material besteht, beispielsweise Siliciumcarbid, in welcher der Strom der Reaktanden auf die erforderliche Reaktionstemperatur erwärmt und eine gleichmäßige Strömung erzeugt wird. Das Festbett kann neben dem Katalysator in üblicher Weise noch inerte Materialien umfassen, um beispielsweise eine Überhitzung des Reaktors zu vermeiden bzw. eine gleichmäßige Strömung zu erreichen. Neben dem Kupferchromitkatalysator kann dem Festbett beispielsweise Siliciumcarbid zugegeben sein. Das inerte Material wird in geeigneter Form in den Reaktor gegeben, beispielsweise in Form eines feinteiligen Granulats, so dass die Strömungsverhältnisse im Reaktor nicht ungünstig beeinflusst werden.

Für die Hydrierung werden das Keton sowie der Wasserstoff dem Reaktor zugeführt, wobei das Keton gegebenenfalls zunächst verdampft wird. Für die Hydrierung wird vorzugsweise ein Druck von mehr als 10 bar, insbesondere bevorzugt innerhalb eines Bereichs von 10 bis 200 bar, besonders bevorzugt 20 bis 50 bar gewählt. Die Temperatur im Reaktor wird vorzugsweise oberhalb von 70°C gewählt, da sonst die Reaktionsgeschwindigkeit stark abnimmt. Besonders bevorzugt wird die Temperatur im Bereich von 70 bis 150°C gewählt. Die Zuführrate des Ketons (LHSV "Liquid Hourly Space Velocity) wird vorzugsweise größer als 5, insbesondere bevorzugt zwischen 0,5 und 1,5 gewählt.

Im allgemeinen wird der Reaktionsmischung kein inertes Trägergas zugegeben.

Das erfindungsgemäße Verfahren eignet sich an sich allgemein für die Reduktion von Ketonen. Es wurde dabei gefunden, dass neben der Ketogruppe vorhandene aromatische Kerne nicht hydriert werden. Vorzugsweise werden beim erfindungsgemäßen Verfahren daher Ketone verwendet, welche zumindest eine aromatische Gruppe umfassen, insbesondere bevorzugt eine Phenylgruppe. Besonders geeignet ist das erfindungsgemäße Verfahren für die Reduktion von Phenylketonen, wobei Acetophenon besonders bevorzugt ist. Die Ketone enthalten bevorzugt keine isolierten Kohlenstoff-Kohlenstoff-Doppelbindungen.

Die Erfindung wird im weiteren unter Bezugnahme auf die beigefügten Figuren näher erläutert. Die Figuren zeigen dabei im einzelnen:

Figur 1: Eine schematische Darstellung eines Festbettreak- tors, wie er im erfindungsgemäßen Verfahren verwendet werden kann;

Figur 2: eine schematische Darstellung einer Vorrichtung zur Durchführung des erfindungsgemäßen Verfahrens;

Figur 3: eine schematische Darstellung eines Verfahrens zur Herstellung eines Katalysators, wie er im erfin- dungsgemäßen Verfahren verwendet wird;

Figur 4: eine Graphik, in welcher die Umwandlungsrate bei der Hydrierung von Acetophenon in Abhängigkeit von der Betriebszeit für verschiedene Katalysatoren dargestellt ist;

Figur 5: eine Graphik, in welcher die Umwandlungsrate bei der Hydrierung von Acetophenon in Abhängigkeit von der Temperatur für zwei Katalysatoren dargestellt ist;

Figur 6: eine Graphik, in welcher die Selektivität bei der Reduktion von Acetophenon in Abhängigkeit von der Temperatur für zwei Katalysatoren dargestellt ist;

Figur 7: eine Graphik, in welcher die Selektivität für die Reduktion von Acetophenon in Abhängigkeit von der Betriebszeit für einen Vergleichskatalysator darge- stellt ist;

Figur 8: eine Graphik, in welcher die Ausbeute an Methylben- zol in Abhängigkeit von der Temperatur für zwei Katalysatoren dargestellt ist.

Figur 1 zeigt beispielhaft einen Schnitt durch einen Festbettreaktor, wie er im erfindungsgemäßen Verfahren verwendet werden kann. Im röhrenförmigen Reaktor 1 ist auf einem Gitter 2 ein Festbett 3 angeordnet, welches den Katalysator in Form von Tabletten, die einen Durchmesser von 3 mm sowie eine Höhe von 3 mm aufweisen, sowie Siliciumcarbid mit einem Partikeldurchmesser von etwa 200 $\mu$m im Verhältnis 1:2 umfasst. Oberhalb des Festbettes 3 ist ein Vorbett 4 angeordnet, welches aus Siliciumcarbidpartikeln mit einem mittleren Durchmesser von etwa 200 $\mu$m gebildet ist. Der Reaktor ist in eine obere Heizzone 5, eine mittlere Heizzone 6 sowie eine untere Heizzone 7 aufgeteilt. In den Heizzonen wird die Temperatur innerhalb des Reaktors 1 so geregelt, dass über die Längsausdehnung des Reaktors 1 annähernd isotherme Bedingungen herrschen. Die axiale Temperaturverteilung im Reaktor 1 kann mittels eines Thermoelements 8 gemessen werden, über welches mittels einer entsprechenden Steuerung die Wärmezufuhr in den Heizzonen 5, 6 und 7 gesteuert wird. Am Kopf des Reaktors 1 wird ein Eduktstrom 9 zugeführt, welcher Wasserstoff sowie gasförmiges Acetophenon enthält. Der Eduktstrom 9 gelangt zunächst in die Vorwärmzone 5, wo im Vorbett 4 eine gleichmäßige Temperatur sowie ein gleichmäßiges Strömungsprofil eingestellt wird. Der Eduktstrom 9 durchläuft dann das Festbett 3, wo eine Reduktion des Acetophenons zum Methylphenylcarbinol erfolgt. Der Produktstrom wird schließlich zur Unterseite des Reaktors 1 herausgeführt.

In Figur 1 ist beispielhaft ein Reaktor mit absteigendem Produktstrom dargestellt. Es ist aber auch möglich, den Reaktor in aufsteigender Fahrweise zu betreiben.

Figur 2 zeigt schematisch einen Aufbau, wie er zur Durchführung des erfindungsgemäßen Verfahrens verwendet werden kann. Über Massendurchflussregler 10 werden Wasserstoffgas 11 sowie gegebenenfalls ein Inertgas 12 einem Vorerhitzer 13 zugeführt, in welchem die Gase aufgeheizt werden. Die Gasaufbereitung umfasst ferner ein Sicherheitsventil 14 sowie ein Manometer 15. Das erwärmte Gas wird über die Zuleitung 16 dem Strom des Acetophenons zugeführt. Dazu wird das Acetophenon aus einem Vorratsbehälter 17 mittels einer Pumpe 18 über eine Zuleitung 19 gemeinsam mit dem Strom der Reaktionsgase einem Verdampfer 20 zugeführt. Das Gemisch der Reaktanten wird anschließend dem Reaktor 1 zugeführt, in welchem die Reduktion des Acetophenons zu Methylphenylcarbinol erfolgt. Der Produktstrom wird einem Kühler 21 zugeleitet, in welchem das Methylphenylcarbinol kondensiert und anschließend in einem Auffangbehälter 22 gesammelt wird. Noch im Produktstrom enthaltenes Wasserstoffgas sowie gegebenenfalls Inertgas wird über die Gasableitung 23 abgeführt.

In Figur 3 ist schematisch die Herstellung des im erfindungsgemäßen Verfahren verwendeten Katalysators dargestellt. In einem ersten Mischbehälter 24 wird destilliertes Wasser vorgelegt und darin $CrO_3$ gelöst. Zur Chromsäurelösung wird anschließend eine Ammoniaklösung zugegeben bis der pH-Wert im Bereich von etwa 9,5 bis 10 liegt. Gegebenenfalls kann die Lösung noch mit destilliertem Wasser verdünnt werden. Die auf 60°C aufgeheizte Lösung wird über eine Pumpe 25 einem zweiten Mischbehälter 26 zugeleitet, in welchem eine wässrige Lösung von Kupfernitrat und Bariumnitrat vorgelegt ist, welcher Siliciumdioxid zugegeben ist. Die Mischung wird über Rührwerk 27 bewegt. Die Temperatur im zweiten Mischbehälter 26 wird über Thermometer 28 und der pH-Wert über das pH-Meter 29 überwacht. Zum Ende der Zugabe beträgt der pH-Wert etwa 6,6 bis 7,0. Bei Bedarf kann der pH durch Zugabe von Ammoniak oder Salpetersäurelösung eingestellt werden. Die Suspension wird zur Alterung noch für ca. 2 Stunden im zweiten Mischbehälter 26 belassen und dann mittels Pumpe 30 der Filterpresse 31 zugeführt, in welcher das Filtrat abgetrennt und über Ableitung 32 dem Abwasser zugeführt wird. Der Filterkuchen wird der Filterpresse 31 entnommen, im Mischbehälter 33 homogenisiert und anschließend über Pumpe 34 einem Vorratsbehälter 35 zugeführt. Aus dem Vorratsbehälter 35 wird

die Suspension über eine Pumpe 37 schließlich einem Sprühtrockner 36 zugeleitet und das anhaftende Wasser durch Zufuhr von Heißluft 38 verdampft. Die festen Bestandteile werden im Zyklon 39 abgetrennt und einem Drehrohrofen 40 zugeführt, in welchem eine Calcinierung durchgeführt wird. Das calcinierte Pulver wird anschließend siebgranuliert, mit Graphit gemischt, kompaktiert, erneut siebgranuliert und in einer Rundlaufpresse tablettiert (nicht dargestellt).

Beispiel 1

Herstellung des Katalysators

In einem ersten Mischbehälter werden 800 kg $CrO_3$ in 2000 kg destilliertem Wasser gelöst und dann eine Ammoniaklösung, welche 410 kg $NH_3$ enthält, während einer Stunde zudosiert. Die Lösung wird mit destilliertem Wasser auf 7000 1 ergänzt und auf 60°C aufgeheizt. Der pH-Wert der Lösung liegt bei 9,5 bis 10. In einem zweiten Mischbehälter wird eine Kupfer- und Bariumnitratlösung, welche 640 kg Kupfer und 150 kg Barium enthält, hergestellt. Die Lösung wird mit destilliertem Wasser auf 5800 1 eingestellt und auf 60°C aufgeheizt. Der pH-Wert dieser Lösung liegt bei 2 bis 2,5. Zu dieser Lösung werden noch 176 kg $SiO_2$ zugemischt. Die im ersten Mischbehälter hergestellte Ammoniumchromatlösung wird während 120 Minuten bei 60°C in den zweiten Mischbehälter zudosiert. Der pH-Wert der Mischung steigt am Ende der Fällung auf 6,6 bis 7,0. Bei Bedarf kann der pH-Wert durch Zugabe von Ammoniak oder Salpetersäurelösung eingestellt werden. Der Niederschlag wird noch für zwei Stunden gealtert und dann die Suspension mit Hilfe einer Filterpresse filtriert. Der Filterkuchen wird in einem Mischbehälter homogenisiert und anschließend sprühgetrocknet und in einem Drehrohrofen calciniert. Das calcinierte Produkt hat eine BET-Oberfläche von 70 $m^2/g$, einen Kupfergehalt von 38%, einen Chromgehalt von 25% und einen $SiO_2$-Gehalt von 10%. Der Glühverlust bei 1000°C beträgt 6%. Das calcinierte Pulver wird siebgranuliert und mit 2% Graphit gemischt. Die Mischung wird kompaktiert, erneut siebgranuliert und mit einer Rundlaufpresse auf 3 x 3 mm tablettiert.

Beispiel 2

In der gleichen Weise wie in Beispiel 1 beschrieben wurde ein Katalysator hergestellt, welcher als Promotor neben Barium noch Mangan enthielt. Das Mangan wurde eingeführt, indem ein Teil des Bariumnitrats durch Mangannitrat ersetzt wurde. Der Katalysator enthielt 33,6% Kupfer, 29,5% Chrom, 1,9% Barium, 2,1% Mangan sowie 9,4% $SiO_2$. Der Glühverlust bei 1000°C wurde zu 3,9% ermittelt.

Die chemische Zusammensetzung und die physikalischen Eigenschaften der erfindungsgemäßen Katalysatoren (Beispiel 1 und Beispiel 2) sowie einiger handelsüblicher Vergleichskatalysatoren (T-4492 = VB 1, T-2130 = VB 2 und T-4489 = VB 3) (Hersteller Süd-Chemie AG) sind in der nachstehenden Tabelle I angegeben.

Tabelle I

| Produkt | | Beisp.1 | Beisp.2 | VB 1 | VB 2 | VB 3 |
|---|---|---|---|---|---|---|
| Teilchenform | | Tabletten | Tabletten | Tabletten | Tabletten | Tabletten |
| Teilchengröße | | 3 x 3mm | 3 x 3mm | 3 x 3mm | 3 x 3mm | 3 x 3mm |
| Chem. Zusammensetzg. | Kupfer (GVfrei) [%] | 38,4 | 33,6 | 34,5 | 25,8 | 44,5 |
| | Zink (GVfrei) [%] | - | - | 43,1 | 53,7 | - |
| | Chrom (GVfrei) [%] | 24,8 | 29,5 | - | - | - |
| | Barium (GVfrei) [%] | 6,1 | 1,9 | - | - | - |
| | Aluminium (GVfrei) [%] | - | - | - | - | 18,0 |
| | Mangan (GVfrei)[%] | - | 2,1 | 2,2 | - | 6,6 |
| | $SiO_2$ (GVfrei) [%] | 10,0 | 9,4 | - | - | - |
| Physikalische Eigenschaften | GV (1000°C)[1]) [%] | 6,2 | 3,9 | 9,6 | 7,7 | 5,6 |
| | Spezifische Oberfläche[2]) [$m^2$/g] | 74 | 58 | 70 | 61 | 46,8 |
| | Schüttdichte [q/l] | 1450 | 1480 | 1420 | 1450 | 1470 |
| | seitendruckfestigkeit[3]) [$m^2$/g] | 102 | 87 | 83 | 72 | 93 |
| | Porenvolumen[4]) [$mm^3$/g] | 176 | 147 | 210 | 165 | 167 |
| | Porenradienverteilung[5]) [$mm^3$/g] | | | | | |
| | 7500 - 875 nm | 0,0 | 1,9 | 0,9 | 0,5 | 0,0 |
| | 875 - 40 nm | 29,9 | 11,8 | 18,3 | 6,2 | 4,6 |
| | 40 - 7 nm | 113,7 | 114,6 | 167,4 | 62,3 | 135,3 |
| | 7 - 3.7 nm | 32,8 | 19,0 | 23,3 | 31,0 | 27,5 |

[1]) GV = Glühverlust
[2]) BET-Oberfläche nach DIN 66131
[3]) Seitendruckfestigkeit nach DIN EN 1094-5
[4]) Porenvolumen nach DIN 66133 (durch Quecksilber)
[5]) Porenradienverteilung nach DIN 6133

Beispiel 3

Hydrierung von Acetophenon

Die in Tabelle 1 aufgeführten Katalysatoren wurden in einer Testanordnung ausgewertet, wie sie schematisch in den Fig. 1 und 2 dargestellt ist. Als Inertgas wurde Stickstoff verwendet.

Die in ihrer oxidierten Form vorliegenden Katalysatoren wurden jeweils zusammen mit SiC im Verhältnis 1:2 in den Reaktor eingefüllt und zunächst 16 Stunden mit reduzierendem Gas (2% $H_2$ in 98% $N_2$) und anschließend nochmals 3 Stunden mit reinem Wasserstoff bei einer Fließgeschwindigkeit von etwa 35 Nl/h reduziert. Die Temperatur im Reaktor 1 wurde auf 175 bzw. 200°C eingestellt. Die Temperatur im Vorerhitzer 13 und im Verdampfer 20 betrug jeweils 150°C. Der Kühler 21 wurde mit Leitungswasser gekühlt. Nach der Reduktion des oxidischen Katalysators wurde vor dem Einführen des Methylethylketons mit stickstoff gespült. Der Druck wurde bis zum Beginn des eigentlichen Tests auf 5 bar Stickstoff eingestellt. Das Gesamtgewicht des Katalysators errechnete sich zu 0,04 Liter x Schüttdichte.

Nach Beendigung der Katalysatoraktivierung wurde der Reaktor 1 vom Druck entlastet und einige Minuten mit Wasserstoff gespült. Die Strömungsgeschwindigkeit des Wasserstoffs betrug 4,8 Nl/$H_2$ (GHSV = 120). Die Strömungsgeschwindigkeit des Methylethylketons betrug 32,2 Nl/h (LHSV = 0,8). Der Druck im Reaktor wurde auf 25 bar eingestellt.

Dann wurde der Wasserstoffdruck auf 35 bar erhöht, und die Temperatur im Vorerhitzer 13, Verdampfer 20, in der Reaktorzuleitung und in den drei Zonen 5, 6, 7 des Reaktors 1 auf 80°C eingestellt. Das Temperaturprofil des Katalysatorbetts wurde alle 10 Minuten gemessen. Nach einer Reaktionszeit von etwa 3 Stunden wurde der Sammelbehälter 22 für die Produkte entfernt, entleert und für die Probenentnahme erneut angeschlossen.

Die Proben wurden jeweils zweimal am Tag (am Morgen und am Nachmittag) entnommen. Dazu wurde zunächst ein Temperaturprofil des Katalysatorbettes aufgenommen. Der Luftdruck, die Raumtemperatur, das Gasvolumen, die Masse des verbrauchten Einsatzmaterials, die Masse der gebildeten Produkte und die Zeit wurden ebenfalls festgehalten. Der Sammelbehälter 22 wurde entleert und anschließend während etwa 1 Stunde das Produkt gesammelt. Die gesammelte Probe wurde anschließend in eine 30ml-schraubflasche überführt. Die Flaschen wurden mit der Probennummer, dem Datum und der Prozesstemperatur beschriftet.

Die flüssigen Proben wurden gaschromatographisch (GC) analysiert wobei ein "Siemens Sichromat 3A"-Gaschromatograph verwendet wurde. Die Säule (30 m lang, Innendurchmesser 0,32 mm; df = 0,50 μm) enthielt CP-Wachs 52 CB. Als Trägergas wurde Wasserstoff mit einem Druck von 0,5 bar verwendet, als Brenngas Luft mit 2 bar und Wasserstoff mit 2 bar. Der "Split" erfolgte bei 135 ml/min. Das Septum wurde mit 13 ml/min gespült. Die Temperatur des Injektors betrug 250°C, die des Detektors (FID) 330°C. Die Probenmenge betrug jeweils 0,1 μl. Die Versuchszeit betrug 60 min, die Temperatur 50°C (0 min) bis 250°C (30 min). Es wurden folgende Peaks identifiziert:

1. N-Decan (int. Standard)
2. Ethylbenzol (EB)
3. Styrol
4. Benzaldehyd
5. Acetophenon
6. 1-Phenylethanol
7. Benzylalkohol
8. 2-Phenylethanol

Die festgehaltenen Parameter bei den Probenahmen und die Ergebnisse der gaschromatographischen Analysen wurden in ein Arbeitsblatt eingetragen, um die MPK-Umwandlung, die MPC-Ausbeute, die MPC-Selektivität und die EB-Ausbeute zu berechnen.

Auswertung

Definitionen (Alle Prozentangaben in Gew.-%)

$$\text{MPK-Umwandlung} = \left[ \left(1 - \frac{\text{MPK [\%](Probe)}}{\text{MPK [\%](Einsatz)}}\right) \right] \times 100$$

$$\text{MPC-Ausbeute} = \frac{\text{MPC[\%](Probe)} \times \text{Flüssigkeit(ab) [g/h]} - \text{MPC[\%](Einsatz)} \times \text{Einsatz(zu) [g/h]} \times 100}{\text{MPK[\%](zu)} \times \text{Einsatz(zu) [g/h]}}$$

$$\text{MPC-Selektivität} = \frac{\text{MPC-Ausbeute}}{\text{MPK-Umwandlung}} \times 100$$

$$\text{EB-Ausbeute} = \frac{\text{EB[\%](Produkte)} - \text{EB[\%](Einsatz)}}{\text{EB[\%](Einsatz)}} \times 100.$$

EB-Ausbeute bei höheren Temperaturen (xy°C) bezogen auf EB-Ausbeute bei 80°C.

$$\frac{\text{EB-Ausbeute[\%](xy°C)} - \text{EB-Ausbeute[\%](80°C)}}{\text{EB-Ausbeute[\%](80°C)}}.$$

Die Testergebnisse sind in den Tabellen II bis VI angegeben und graphisch in den Figuren 4 bis 8 dargestellt.

Tabelle II

| Ergebnisse mit dem Katalysator von Beispiel 1 | | | | | | |
|---|---|---|---|---|---|---|
| Betriebszeit | | MPK-Umwandlung | MPC-Selektivität | Styrol | Benzaldehyd | Ethylbenzol-Produktion |
| | | | | | | |
| [h] | | [%] | [%] | [%] | [%] | [%] |
| | | | | | | |
| 22,50 | | 67,4 | 99,1 | 0,08 | 0,08 | 15,8* |
| 28,75 | | 71,0 | 98,3 | 0,00 | 0,07 | 14,5* |
| 46,50 | | 69,7 | 100,7 | 0,00 | 0,08 | 17,6* |
| 52,20 | | 69,8 | 97,5 | 0,00 | 0,07 | 14,8* |
| 70,20 | | 69,7 | 100,1 | 0,00 | 0,08 | 15,5* |
| 76,70 | | 66,1 | 99,7 | 0,00 | 0,08 | 16,2 |
| 98,50 | | 60,5 | 97,6 | 0,04 | 0,09 | 14,8* |
| 116,00 | | 60,0 | 101,9 | 0,03 | 0,08 | 17,4* |
| 141,25 | | 59,7 | 97,0 | 0,00 | 0,08 | 16,7 |
| 162, 50 | | 60,3 | 97,0 | 0,00 | 0,08 | 16,4* |
| 168,50 | | 57,5 | 94,9 | 0,04 | 0,09 | 15,0* |
| 186,00 | | 62,2 | 95,9 | 0,00 | 0,08 | 15,7* |

(fortgesetzt)

| Ergebnisse mit dem Katalysator von Beispiel 1 | | | | | | |
|---|---|---|---|---|---|---|
| Betriebszeit | | MPK-Umwandlung | MPC-Selektivität | Styrol | Benzaldehyd | Ethylbenzol-Produktion |
| | | | | | | |
| [h] | | [%] | [%] | [%] | [%] | [%] |
| | | | | | | |
| 191,75 | | 60,8 | 96,7 | 0,00 | 0,08 | 16,5* |
| 212,75 | | 59,6 | 97,9 | 0,00 | 0,08 | 17,8* |
| 234,75 | | 60,0 | 96, 8 | 0,00 | 0,08 | 16,1 |
| 240,50 | | 59 | 100,1 | 0,00 | 0,08 | 18,8* |
| 258,50 | | 60,3 | 96,4 | 0,00 | 0,08 | 15,0* |
| | | | | | | |
| | Temperatur | | | | | |
| | | | | | | |
| 306,00 | 100 | 80,2 | 94,3 | - | - | 7,8** |
| 332,25 | 130 | 92,1 | 86, 5 | - | - | 59,8** |
| 353,75 | 150 | 92,3 | 70,1 | - | - | 109,1** |
| 376,75 | 80 | 47,5 | 92,1 | 0,05 | 0,1 | 17,0* |
| * durch Hydrierung von Styrol (Umwandlung von Styrol = Ethylbenzol-Ausbeute)<br>** durch Hydrogenolyse von MPK (Ethylbenzolbildung aus Styrol wird als Gew.-% Ethylbenzol am Ausgang bei 80°C definiert) | | | | | | |

Tabelle III

| Ergebnisse mit dem Katalysator von Beispiel 2 | | | | | |
|---|---|---|---|---|---|
| Betriebszeit | MPK-Umwandlung | MPC-Selektivität | Styrol | Benzaldehyd | Ethylbenzol-Ausbeute |
| | | | | | |
| [h] | [%] | [%] | [%] | [%] | [%] |
| 19,5 | 60,4 | 94,3 | 0 | 0,07 | 14,1* |
| 26,5 | 59,9 | 93,3 | 0 | 0,06 | 13,8* |
| 43,75 | 58,8 | 95 | 0 | 0,06 | 14,7* |
| 50,75 | 58,3 | 97,3 | 0 | 0,06 | 14,1* |
| 68 | 57,6 | 95,5 | 0 | 0,06 | 15,4* |
| * durch Hydrierung von Styrol (Umwandlung von Styrol = Ethylbenzol-Ausbeute) | | | | | |

Tabelle IV

| Ergebnisse mit dem Katalysator von VB1 | | | | | | |
|---|---|---|---|---|---|---|
| Betriebszeit | | MPK-Umwandlung | MPC-Selektivität | Styrol | Benzaldehyd | Ethylbenzol-Ausbeute |
| | | | | | | |
| [h] | | [%] | [%] | [%] | [%]. | [%] |
| | | | | | | |
| 6,0 | | 86,6 | 95,6 | 0,05 | 0,11 | 19,7* |
| 26,25 | | 80,4 | 97,9 | 0 | 0,07 | 16,5* |
| 33,25 | | 78,4 | 96,0 | 0 | 0,07 | 15,2* |
| 50,75 | | 74,4 | 99,3 | 0 | 0,07 | 16,4* |
| 56,75 | | 73,6 | 96,4 | 0 | 0,07 | 15,7 |
| 74,50 | | 71,6 | 99,7 | 0 | 0,07 | 17,3* |
| 79,75 | | 69,7 | 96,4 | 0 | 0,07 | 13,1* |
| 96,25 | | 70,2 | 99,3 | 0 | 0,07 | 16,7* |
| 103,00 | | 69,2 | 97,9 | 0 | 0,07 | 14, 3* |
| 122,00 | | 66,7 | 100,3 | 0 | 0,08 | 17, 6* |
| 143,73 | | 64,7 | 98,0 | 0 | 0,08 | 14,7* |
| 168,06 | | 63,7 | 98,1 | 0 | 0,07 | 16,2* |
| 173,06 | | 64,4 | 99,5 | 0 | 0,08 | 17,6* |
| 193,30 | | 63,6 | 98,5 | 0 | 0,07 | 16,0* |
| 198,30 | | 62,3 | 96,2 | 0 | 0,08 | 15,9* |
| 216,30 | | 62,1 | 99,1 | 0 | 0,07 | 16,6* |
| 221,30 | | 62,6 | 99,3 | 0 | 0,08 | 14,9* |
| 239,30 | | 61,2 | 100,5 | 0 | 0,08 | 16,8* |
| 243,80 | | 61,8 | 99,4 | 0 | 0,08 | 17,4* |
| 266,05 | | 61,6 | 99,6 | 0 | 0,08 | 17,6* |
| | | | | | | |
| | | | | | | |
| | Temperatur | | | | | |
| | | | | | | |
| 309,80 | 100 | 87,3 | 94,4 | - | - | 5,6** |
| 337,55 | 130 | 98 | 79,7 | - | - | 82,3** |
| 358,55 | 150 | 93,2 | 67,3 | - | - | 128,0** |
| 378,05 | 80 | 53,6 | 93,4 | 0,05 | 0,1 | 22,6* |
| * durch Hydrierung von Styrol (Umwandlung von Styrol = Ethylbenzol-Ausbeute)<br>** durch Hydrogenolyse von MPK (Ethylbenzolbildung aus Styrol wird als Gew.-% Ethylbenzol am Ausgang bei 80°C definiert) | | | | | | |

Tabelle V

| Ergebnisse mit dem Katalysator von VB 2 (T-2130) | | | | | |
|---|---|---|---|---|---|
| Betriebszeit | MPK-Umwandlung | MPC-Selektivität | Styrol | Benzaldehyd | Ethylbenzol-Ausbeute |
| | | | | | |
| [h] | [%] | [%] | [%] | [%] | [%] |
| | | | | | |
| 24,5 | 83,8 | 100,1 | 0 | 0,06 | 17,6* |
| 31,25 | 82,0 | 97,4 | 0 | 0,06 | 18,0* |
| 49,00 | 81,8 | 98,3 | 0 | 0,06 | 18,8* |
| 55,00 | 78,2 | 99,3 | 0 | 0,06 | 19,2* |
| 73,80 | 74,6 | 97,2 | 0 | 0,06 | 16,3* |
| 102,30 | 70,5 | 95,5 | 0 | 0,06 | 14,1* |
| 123,40 | 67,6 | 98,1 | 0 | 0,06 | 15,9* |
| 144,90 | 54,6 | 94,4 | 0 | 0,06 | 15,2* |
| 168,70 | 54,6 | 96,0 | 0 | 0,06 | 15,9* |
| 193,40 | 52,9 | 95,1 | 0 | 0,06 | 15,4* |
| * durch Hydrierung von Styrol (Umwandlung von Styrol = Ethylbenzol-Ausbeute) | | | | | |

Tabelle VI

| Ergebnisse mit dem Katalysator von VB 3 (T-4489) | | | | | |
|---|---|---|---|---|---|
| Betriebszeit | MPK-Umwandlung | MPC-Selektivität | Styrol | Benzaldehyd | Ethylbenzol-Ausbeute |
| | | | | | |
| [h] | [%] | [%] | [%] | [%] | [%] |
| | | | | | |
| 25,0 | 64 | 99,1 | 0 | 0,07 | 19,1* |
| 31,3 | 63,8 | 95,0 | 0 | 0,07 | 15,4* |
| 49,5 | 59,4 | 98,8 | 0 | 0,07 | 16,8* |
| 56,0 | 58,3 | 97,8 | 0 | 0,07 | 16,2* |
| 75,5 | 55,8 | 96,1 | 0 | 0,07 | 15,5* |
| * durch Hydrierung von Styrol (Umwandlung von Styrol = Ethylbenzol-Ausbeute) | | | | | |

Figur 4 zeigt eine Graphik, in welcher die Umwandlungsrate des Acetophenon in Abhängigkeit von der Betriebszeit dargestellt ist. Die Katalysatoren aus den Vergleichsbeispielen 1 und 2 zeigen zu Beginn des Versuches eine hohe Umwandlungsrate, die beim Katalysator VB 1 mit zunehmender Betriebszeit kontinuierlich abnimmt. Der Katalysator VB 2 zeigt anfänglich einen ähnlichen Verlauf wie der Katalysator VB 1, wobei jedoch nach einer Betriebszeit von 140 Stunden ein scharfer Abfall der Umwandlungsrate beobachtet wurde. Nach Ablauf der Versuchszeit von 200 Stunden wurden die Katalysatoren aus dem Reaktor entfernt, wobei sich zeigte, dass mehr als 50% der aus dem Katalysator VB 2 hergestellten Tabletten zerfallen waren und nur noch als Pulver vorlagen. Der im erfindungsgemäßen Verfahren verwendete Katalysator aus Beispiel 1 zeigte zwar zu Beginn der Versuchszeit eine etwas niedrigere Umwandlungsrate als die Katalysatoren VB 1 und VB 2. Mit zunehmender Betriebszeit näherte sich die Umwandlungsrate jedoch an die Umwandlungsrate an, wie sie mit dem Katalysator VB 1 beobachtet wurde.

Die Umwandlungsraten für den Katalysator VB 3 sowie den Katalysator aus Beispiel 2 waren zu Beginn des Versuchs im Vergleich zu den anderen gestesteten Katalysatoren vergleichsweise niedrig, so dass der Test nach ca. 70 Stunden abgebrochen wurde.

In den folgenden Figuren sind nur noch die Katalysatoren aus Beispiel 1 und Vergleichsbeispiel 1 berücksichtigt, da sie in Bezug auf die Umwandlungsrate in Abhängigkeit von der Betriebszeit das beste Verhalten zeigten.

In Figur 5 ist die Umwandlungsrate in Abhängigkeit von der Temperatur dargestellt. Man erkennt, dass sowohl für den Katalysator VB 1 wie auch für den Katalysator aus Beispiel 1 bei Temperaturen im Bereich von 80°C eine Umwandlungsrate von ca. 60% beobachtet wird. Mit steigender Temperatur nimmt die Umwandlungsrate für das Acetophenon zu, bis sie sich bei ca. 130°C einem Plateau nähert. Die beiden Kurven zeigen dabei einen annähernd parallelen Verlauf.

Figur 6 zeigt die Selektivität in Bezug auf Methylphenylcarbinol in Abhängigkeit von der Temperatur. Es zeigt sich, dass der Katalysator aus VB 1 bei niedrigen Temperaturen eine geringfügig höhere Selektivität aufweist als der im erfindungsgemäßen Verfahren verwendete Katalysator aus Beispiel 1. Mit steigender Temperatur nimmt die Selektivität für den Katalysator aus VB 1 stärker ab. Bei Temperaturen oberhalb von ca. 120°C zeigt der Katalysator aus Beispiel 1 im Vergleich zum Katalysator VB 1 eine bessere Selektivität.

In Figur 7 ist für den Katalysator aus Beispiel 1 sowie VB 1 die Selektivität in Abhängigkeit von der Betriebszeit angegeben. Es zeigt sich, dass für den Katalysator aus Beispiel 1 die Selektivität über die Betriebszeit hinweg annähernd konstant bleibt. Für den Katalysator aus VB1 sinkt die Selektivität mit zunehmender Betriebszeit leicht ab.

In Figur 8 ist schließlich die Ausbeute an Ethylbenzol als unerwünschtes Nebenprodukt in Abhängigkeit von der Temperatur dargestellt. Während bei niedrigen Temperaturen beide Katalysatoren nur geringe Mengen an Ethylbenzol erzeugen, wird bei höheren Temperaturen beim im erfindungsgemäßen Verfahren verwendeten Katalysator aus Beispiel 1 ein deutlich niedriger Anteil an Nebenprodukt Ethylbenzol beobachtet.

Insgesamt ergibt sich, dass bei Bedingungen, wie sie für die industrielle Anwendung verwendet werden, der im erfindungsgemäßen Verfahren verwendete Katalysator eine bessere Selektivität und damit einen geringeren Anteil unerwünschter Nebenprodukte ergibt, als der als Vergleichsbeispiel herangezogene Katalysator VB 1.

Seitendruckfestikgkeit

Die Katalysatortabletten aus Beispiel 1 sowie Vergleichsbeispiel 1 wurden jeweils vor und nach, der in Beispiel 3 durchgeführten Hydrierung auf ihre Seitendruckfestigkeit (DIN EN 1094-5) überprüft. Die Ergebnisse sind in Tabelle VII zusammengefasst.

Tabelle VII

| Prüfung der Seitenfestigkeit | | | | | | |
|---|---|---|---|---|---|---|
| Produkt | | | Beispiel 1 | | vgl. Bsp. 1 | |
| Teilchenform | | | Tabletten | | Tabletten | |
| Teilchengröße | | | 3x3 mm | | 3x3 mm | |
| Art der Tabletten | | | vor Test | nach Test | vor Test | nach Test |
| Seitendruckfestigkeit | Durchschnitt | [N] | 102 | 106 | 83 | 41 |
| | Min. | [N] | 28 | 48 | 46 | 26 |
| | Max | [N] | 197 | 189 | 147 | 69 |

Der erfindungsgemäß verwendete Katalysator aus Beispiel 1 zeigt auch nach dem Test, d.h. nach der Hydrierung von Acetophenon, annähernd keine Veränderung der Seitendruckfestigkeit bzw. wird sogar eine gewisse Steigerung beobachtet. Der Katalysator aus Vergleichsbeispiel 1 zeigt hingegen eine Verschlechterung der Seitendruckfestigkeit. Bei einer industriellen Anwendung bedeutet dies, dass der Zerfall des Katalysators aus Beispiel 1 erst wesentlich später eintritt als beim Katalysator aus Vergleichsbeispiel 1. Damit steigt der Gegendruck im Reaktor über die Zeit wesentlich langsamer an. Es können wesentlich längere Betriebszeiten erreicht werden, d.h. der Katalysator muss erst in wesentlich größeren zeitlichen Abständen ausgetauscht werden.

Bezugszeichenliste

1 Reaktor
2 Gitter
3 Festbett

4 Vorbett
5 obere Heizzone
6 mittlere Heizzone
7 untere Heizzone
8 Thermoelement
9 Produktstrom
10 Massendurchflussregler
11 Wasserstoffgas
12 Inertgas
13 Vorerhitzer
14 Sicherheitsventil
15 Manometer
16 Zuleitung
17 Vorratsbehälter
18 Pumpe
19 Zuleitung
20 Verdampfer

21 Kühler
22 Auffangbehälter
23 Gasableitung
24 erster Mischbehälter
25 Pumpe
26 zweiter Mischbehälter
27 Rührwerk
28 Thermometer
29 pH-Meter
30 Pumpe
31 Filterpresse
32 Ableitung
33 Mischbehälter
34 Pumpe
35 Vorratsbehälter
36 Sprühtrockner
37 Pumpe
38 Heißluftzufuhr
39 Zyklon
40 Drehrohrofen

**Patentansprüche**

**1.** Verfahren zur Hydrierung von Phenylketonen, wobei das Keton im Gemisch mit Wasserstoff einem Katalysatorbett zugeführt wird, welches einen Kupferchromit-Katalysator enthält, der einen Anteil an $SiO_2$ aufweist, wobei der Katalysator Kupfer in einem Anteil von 30 bis 40 Gew.-%, sowie Chrom in einem Anteil von 20 bis 30 Gew.-% enthält, und der Anteil an $SiO_2$ im Bereich von 5 bis 15 Gew.-% gewählt ist, jeweils bezogen auf die oxidierte Form des Katalysators, und wobei der Katalysator bereitgestellt wird, indem wässrige Lösungen der Metallsalze hergestellt und vereinigt werden, sodass ein Niederschlag erhalten wird, wobei zumindest einer der Metallsalzlösungen $SiO_2$ zugegeben ist.

**2.** Verfahren nach Anspruch 1, wobei der Kupferchromit-Katalysator, bezogen auf den oxidierten Katalysator, Promotormetalle in einem Anteil von 1 bis 6 Gew.-% enthält.

**3.** Verfahren nach Anspruch 2, wobei die Promotormetalle ausgewählt sind aus Barium und/oder Mangan.

**4.** Verfahren nach einem der vorhergehenden Ansprüche, wobei der Kupferchromit-Katalysator eine spezifische Oberfläche von etwa 50 bis 80 $m^2/g$ aufweist.

5. Verfahren nach einem der vorhergehenden Ansprüche, wobei das Katalysatorbett als Festbett ausgestaltet ist.

6. Verfahren nach einem der vorhergehenden Ansprüche, wobei die Hydrierung in einem Druckbereich von 10 bis 200 bar, einer Temperatur von mehr als 70°C und einer LHSV im Bereich von 0,5 bis 1,5 durchgeführt wird.

7. Verfahren nach einem der vorhergehenden Ansprüche, wobei dem Wasserstoff und dem Keton kein inertes Trägergas beigegeben ist.

8. Verfahren nach einem der vorhergehenden Ansprüche, wobei das Phenylketon Acetophenon ist.

## Claims

1. Process for the hydrogenation of phenyl ketones, wherein the ketone is fed mixed with hydrogen to a catalyst bed which contains a copper chromite catalyst which contains $SiO_2$, wherein the catalyst contains 30 to 40 wt.-% copper and 20 to 30 wt.-% chrome, and the $SiO_2$ content is chosen in the range from 5 to 15 wt.-%, relative in each case to the oxidized form of the catalyst, and wherein the catalyst is provided by preparing and combining aqueous solutions of the metal salts, with the result that a precipitate is obtained, wherein $SiO_2$ is added to at least one of the metal salt solutions.

2. Process according to claim 1, wherein the copper chromite catalyst contains 1 to 6 wt.-% promoter metals relative to the oxidized catalyst.

3. Process according to claim 2, wherein the promoter metals are selected from barium and/or manganese.

4. Process according to one of the previous claims, wherein the copper chromite catalyst has a specific surface area of approximately 50 to 80 $m^2/g$.

5. Process according to one of the previous claims, wherein the catalyst bed is designed as a fixed bed.

6. Process according to one of the previous claims, wherein the hydrogenation is carried out in a pressure range from 10 to 200 bar, at a temperature of more than 70°C and a LHSV in the range from 0.5 to 1.5.

7. Process according to one of the previous claims, wherein inert carrier gas is not added to the hydrogen and the ketone.

8. Process according to one of the previous claims, wherein the phenyl ketone is acetophenone.

## Revendications

1. Procédé en vue de l'hydrogénation de phénylcétones, la cétone étant alimentée en mélange avec de l'hydrogène à un lit de catalyseur qui contient un catalyseur au chromite de cuivre, qui présente une proportion de SiO2, le catalyseur contenant du cuivre dans une proportion de 30 à 40 % en poids, ainsi que du chrome dans une proportion de 20 à 30 % en poids, et la proportion de SiO2 étant choisie dans le domaine de 5 à 15 % en poids, à chaque fois par rapport à la forme oxydée du catalyseur, et le catalyseur étant mis à disposition par préparation et combinaison de solutions aqueuses de sels métalliques, de manière à obtenir un précipité, du SiO2 étant ajouté à au moins une des solutions de sels métalliques.

2. Procédé selon la revendication 1, le catalyseur au chromite de cuivre contenant, par rapport au catalyseur oxydé, des métaux promoteurs dans une proportion de 1 à 6 % en poids.

3. Procédé selon la revendication 2, les métaux promoteurs étant sélectionnés parmi le baryum et/ou le manganèse.

4. Procédé selon l'une quelconque des revendications précédentes, le catalyseur au chromite de cuivre présentant une surface spécifique d'environ 50 à 80 $m^2/g$.

5. Procédé selon l'une quelconque des revendications précédentes, le lit de catalyseur étant réalisé sous la forme d'un lit fixe.

6. Procédé selon l'une quelconque des revendications précédentes, l'hydrogénation étant effectuée dans un domaine de pression de 10 à 200 bars, à une température de plus de 70°C et à une vitesse LHSV dans le domaine de 0,5 à 1,5.

7. Procédé selon l'une quelconque des revendications précédentes, aucun gaz vecteur inerte n'étant ajouté à l'hydrogène et à la cétone.

8. Procédé selon l'une quelconque des revendications précédentes, la phénylcétone étant l'acétophénone.

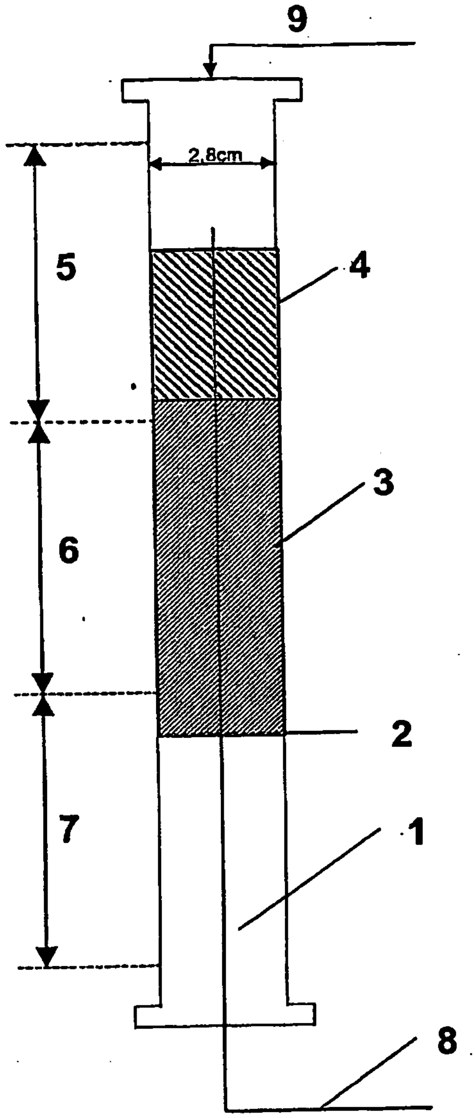


Fig. 1

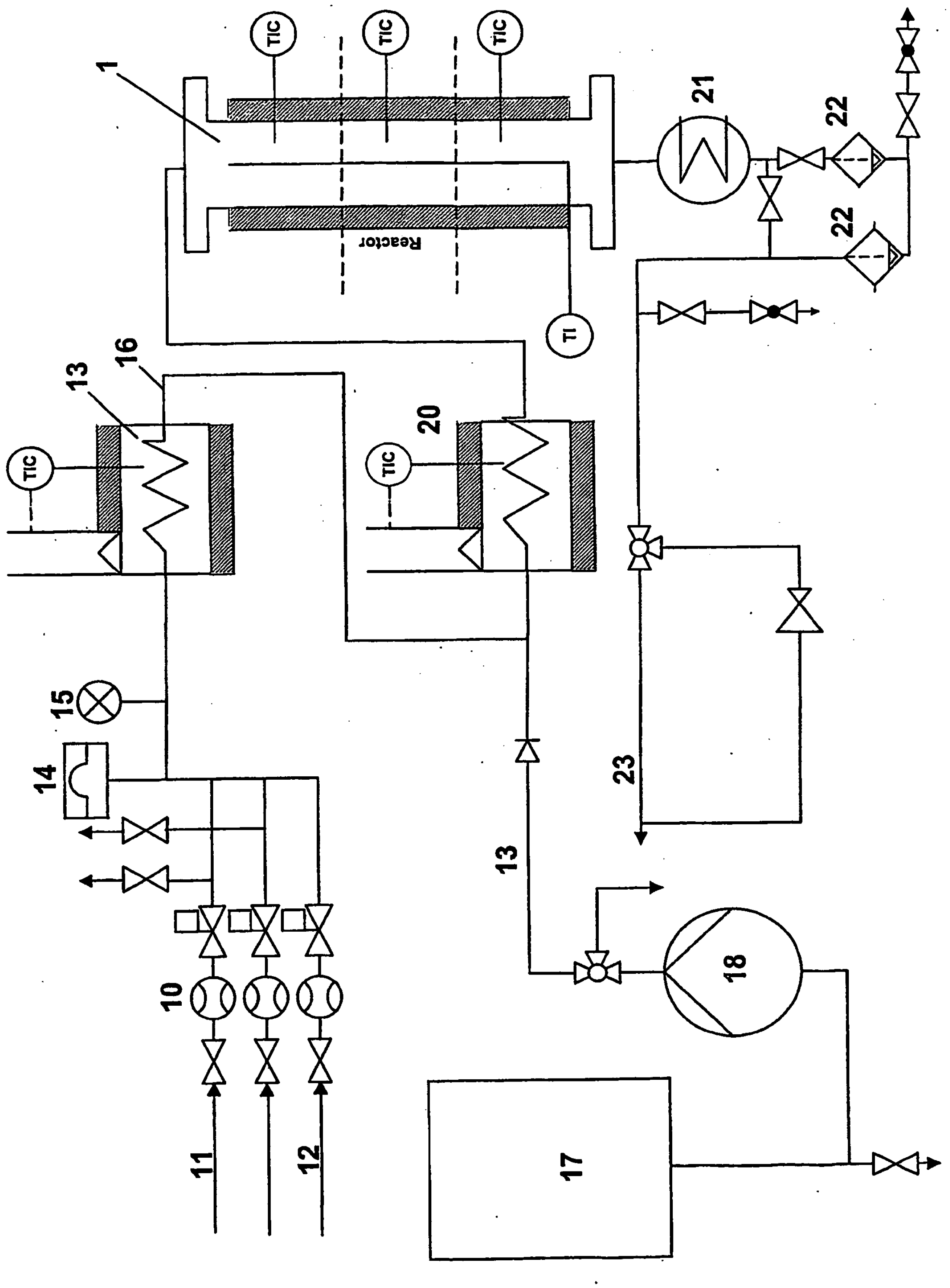
1
TIC
TIC
TIC
Reactor
TI
21
22
22
16
13
TIC
20
TIC
15
14
23
13
10
11
12
18
17

Fig. 2

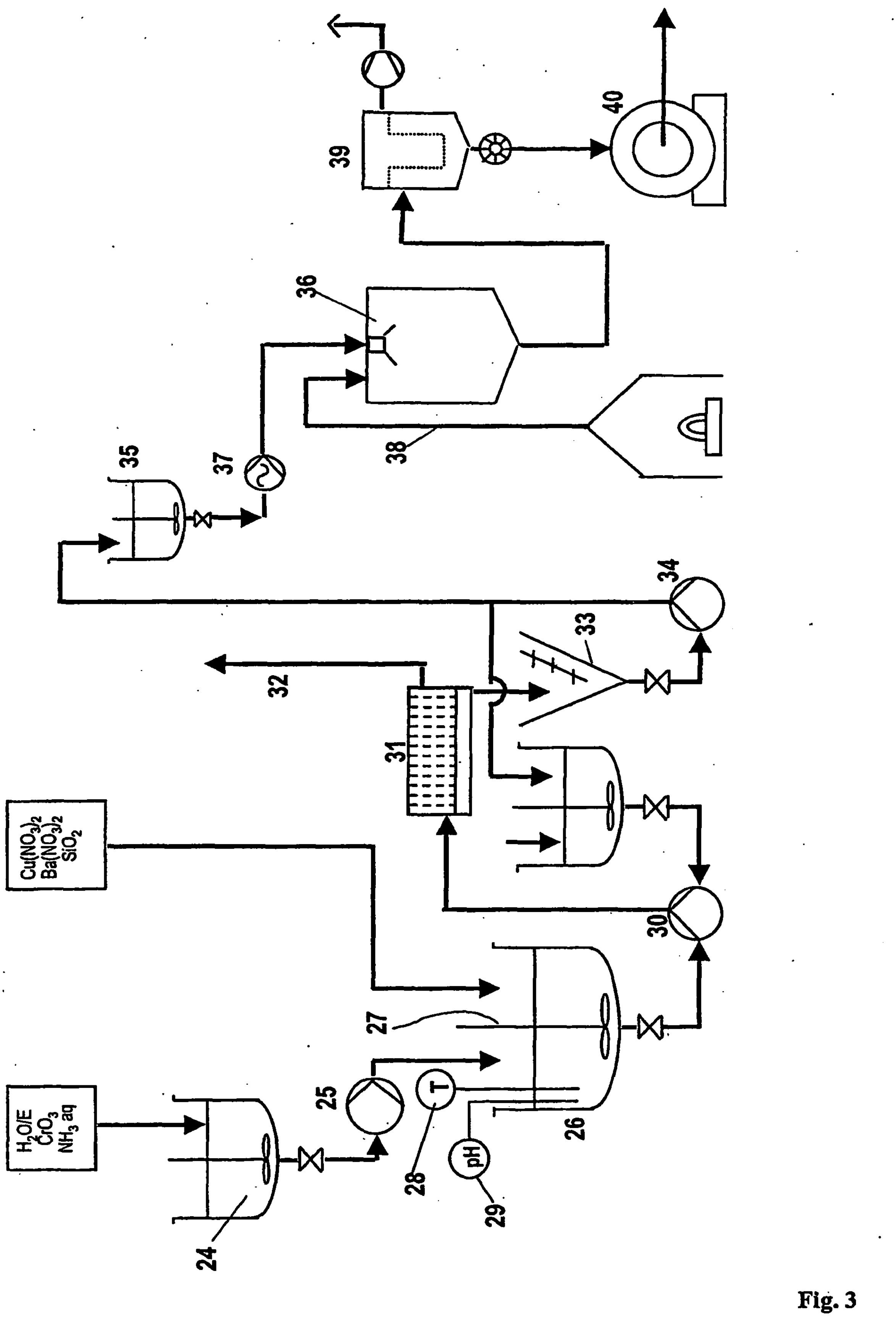
H2O/E
CrO3
NH3 aq
Cu(NO3)2
Ba(NO3)2
SiO2
T
pH
24
25
26
27
28
29
30
31
32
33
34
35
36
37
38
39
40

Fig. 3

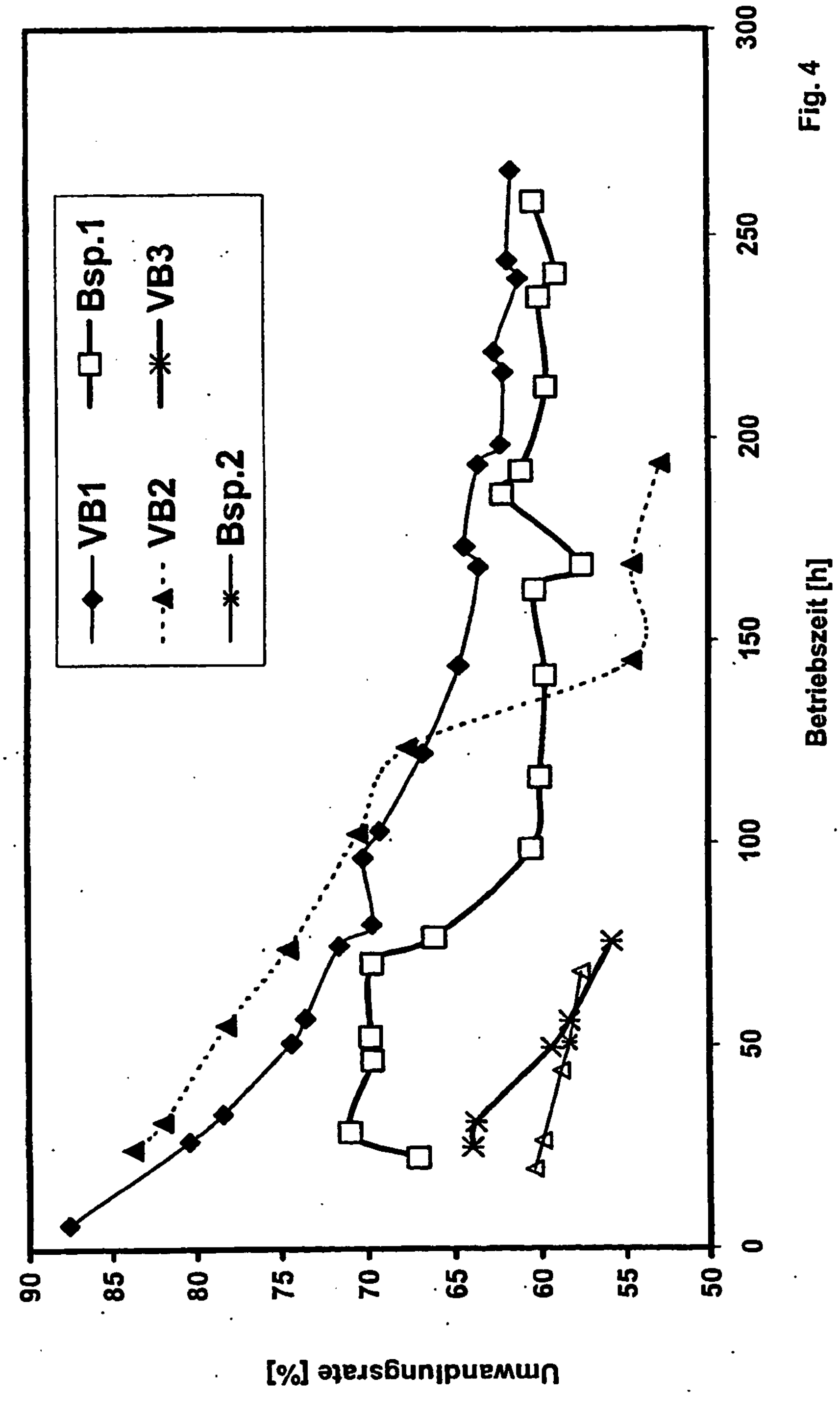
VB1
VB2
Bsp.2
Bsp.1
VB3
Umwandlungsrate [%]
90
85
80
75
70
65
60
55
50
0
50
100
150
200
250
300
Betriebszeit [h]
Fig. 4

Fig. 4

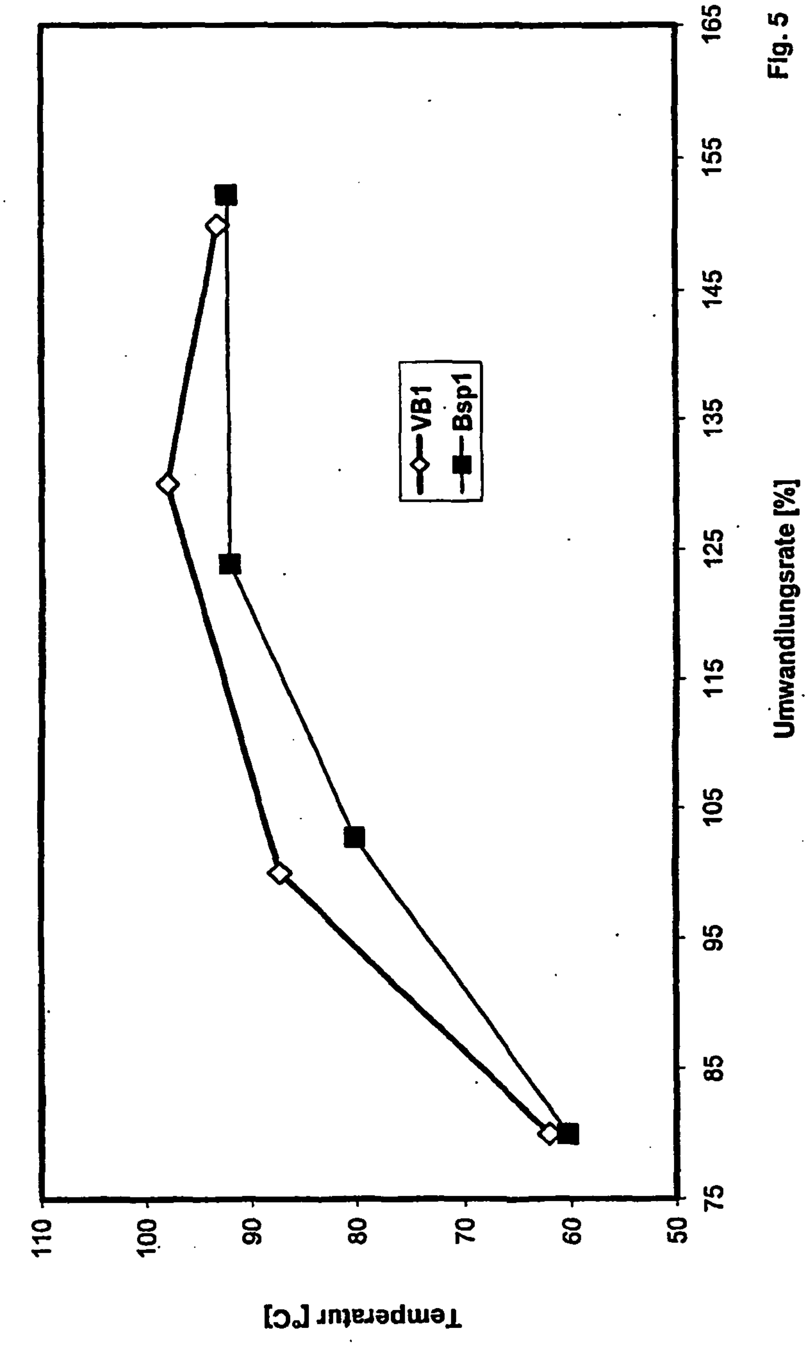

Temperatur [°C]
110
100
90
80
70
60
50
75
85
95
105
115
125
135
145
155
165
Umwandlungsrate [%]
VB1
Bsp1
Fig. 5


Fig. 5

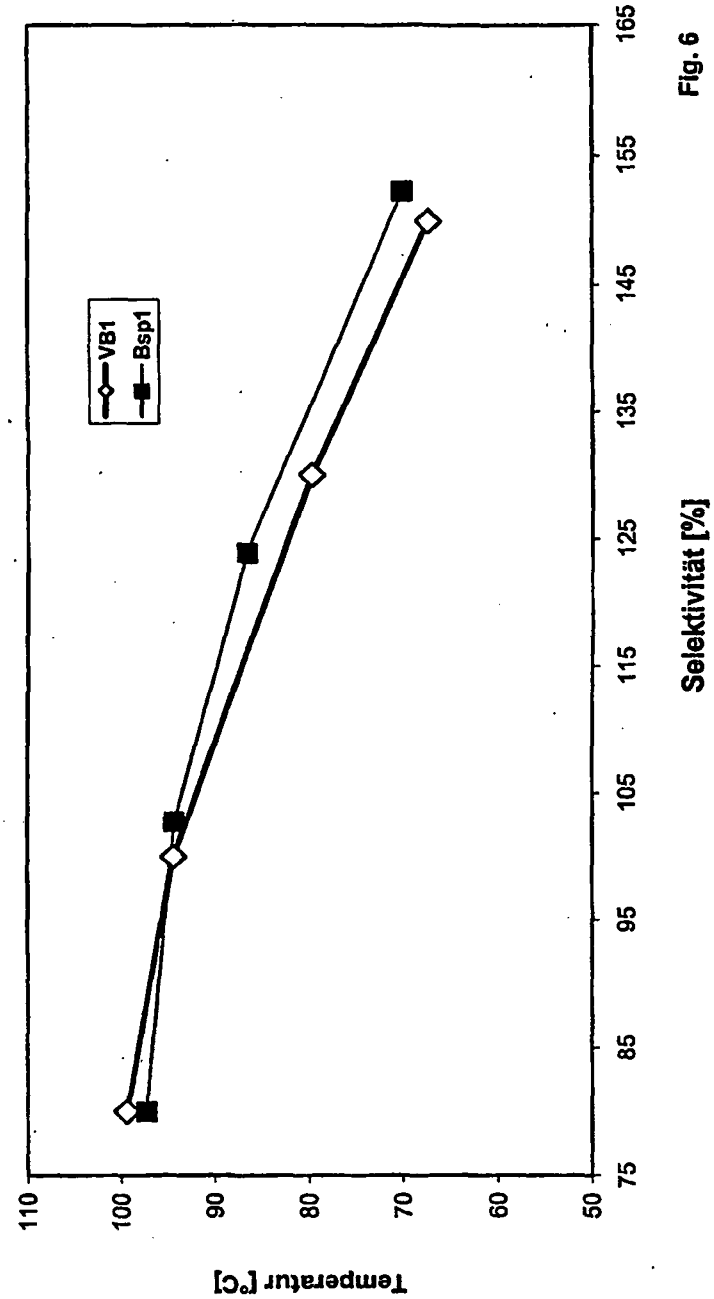
Temperatur [°C]
110
100
90
80
70
60
50
75
85
95
105
115
125
135
145
155
165
Selektivität [%]
VB1
Bsp1
Fig. 6

Fig. 6

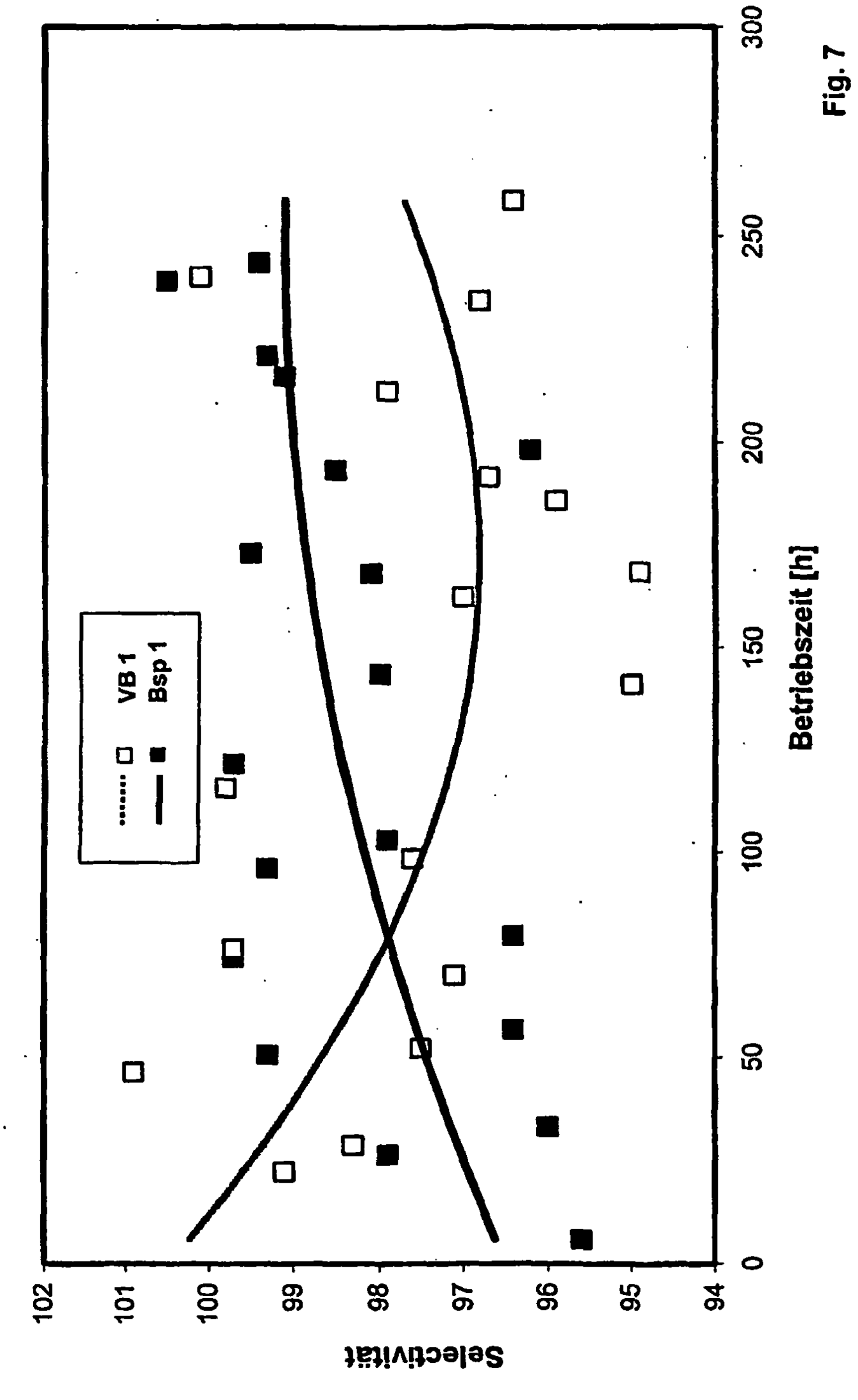
Selectivität
102
101
100
99
98
97
96
95
94
0
50
100
150
200
250
300
Betriebszeit [h]
VB 1
Bsp 1
Fig. 7

Fig. 7

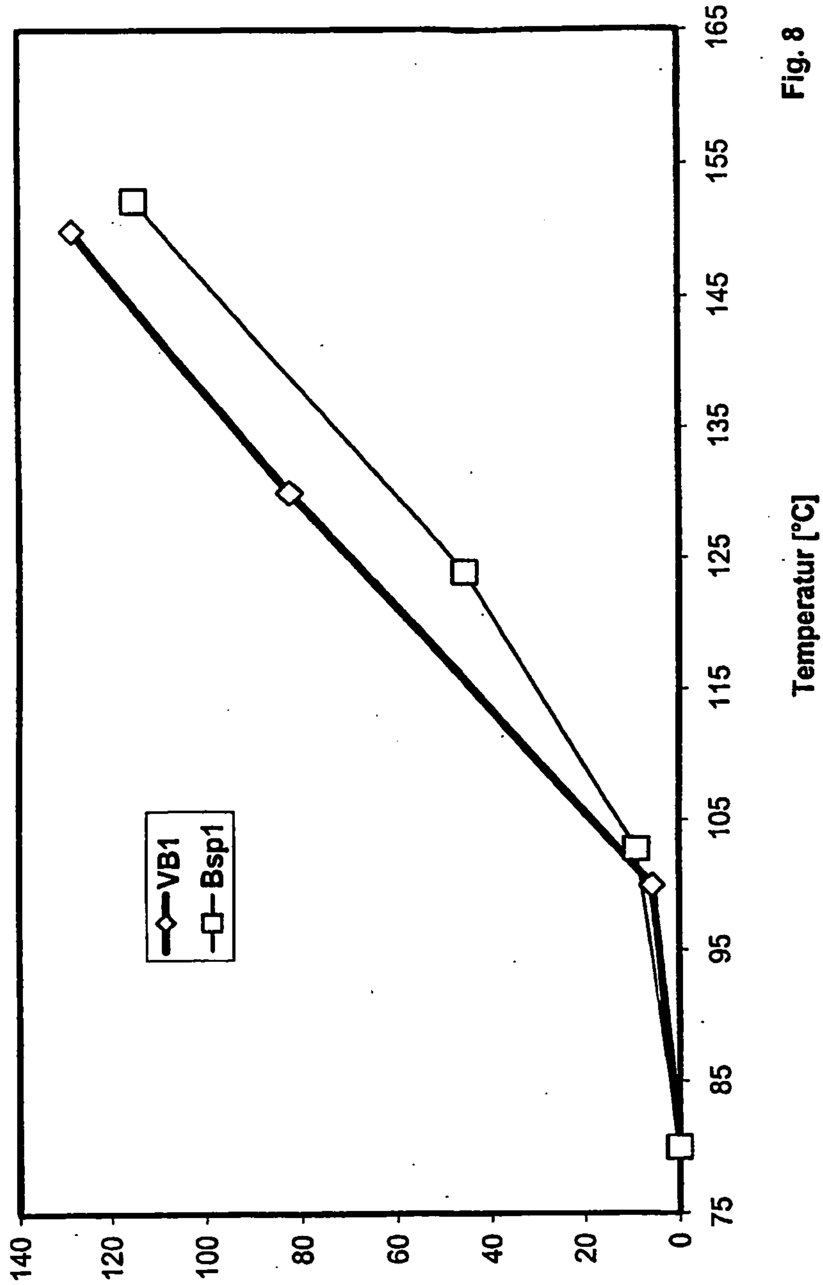
VB1
Bsp1
EB-Ausbeute [%]
0
20
40
60
80
100
120
140
Temperatur [°C]
75
85
95
105
115
125
135
145
155
165
Fig. 8

Fig. 8

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- *Journal of Organic Chemistry,* 1949, vol. 14, 1089-1093